# EUROPEAN PATENT SPECIFICATION

(11) **EP 2 131 923 B1**
(45) Date of publication and mention of the grant of the patent: **01.08.2012**
(21) Application number: 07747967.3
(22) Date of filing: 27.03.2007
(51) Int. Cl.: A61N 1/372

(54) **METHOD AND SYSTEM FOR INITIATING COMMUNICATION BETWEEN A HOME MONITORING DEVICE AND AN IMPLANTABLE MEDICAL DEVICE**
VERFAHREN UND SYSTEM ZUR EINLEITUNG EINER KOMMUNIKATION ZWISCHEN EINEM HEIMÜBERWACHUNGSGERÄT UND EINEM IMPLANTIERBAREN MEDIZINPRODUKT
PROCÉDÉ ET SYSTÈME D'INITIALISATION D'UNE COMMUNICATION ENTRE UN DISPOSITIF DE SURVEILLANCE DOMESTIQUE ET UN DISPOSITIF MÉDICAL IMPLANTABLE

(43) Date of publication of application: 16.12.2009
(73) Proprietor: St. Jude Medical AB, 175 84 Järfälla (SE)
(72) Inventor: LYCHOU, Leif, S-163 45 Spånga (SE); LJUNGSTRÖM, Jan, S-165 74 Hässelby (SE); SKÖLDENGEN, Niklas, S-187 50 Täby (SE); ABRAHAMSON, Hans, S-113 38 Stockholm (SE)
(86) International application number: PCT/SE2007/000296
(87) International publication number: WO 2008/118045

(56) References cited:
- EP-A1- 1 174 163
- WO-A1-2005/099817
- WO-A1-2005/099817
- US-A1- 2003 114 898
- US-A1- 2003 114 898
- US-A1- 2003 149 459
- US-A1- 2003 229 383

## Description

### Technical field of the Invention

The present disclosure relates to a method and system for establishing a wireless communication session between an implantable medical device and a home monitoring device. The invention is set out in the appended claims.

### Background Art

When communicating with an implantable medical device (IMD) implanted in tissue of a human or animal body, a radio frequency (RF) transceiver is used for wireless communication of signals to/from RF communication circuitry comprised in the IMD. An IMD may be embodied in the form of an implantable cardioverter defibrillator (ICD), a neural simulator, an implantable drug pump, an implantable cardiac monitor or health monitor, a pacemaker, etc. Communication may be established with the IMD for a number of reasons. For instance, medical personnel may want to monitor and/or adjust parameters of the IMD, it may be desirable to perform medical or therapeutic treatment of the body via the IMD, e.g. defibrillation, restart of a stopped heart, injection of an insulin dose, etc. Needless to say, RF signaling enables establishment of a communication channel without having to open up the patient surgically. In a home environment, the patient may have a monitoring device employed to establish RF communication with transceiver circuitry within an IMD. From a user's point of view, a home monitoring device may be viewed upon as a computer arranged with an RF transceiver, a user interface for controlling the monitoring device and dedicated software designed for the purpose of communicating with an IMD. When establishing communication with an IMD, the monitoring device broadcasts a wake-up signal to which the IMD responds before a communication session may be undertaken between the monitoring device and the IMD, i.e. before payload data is exchanged between the monitoring device and the IMD.

When an IMD detects a wake-up signal, initiates communication and engages in a communication session with the monitoring device, a significant amount of energy is consumed at the IMD, since its RF transceiver (and associated RF electronics) exits low-power mode and enters an active communication mode.

US 7,110,823 generally discloses establishment of communication between an implant device and an external device via an RF link. In order to conserve power, the RF telemetry system of the implant is only activated periodically with the period of activation being sufficiently short to allow a reasonably prompt response to a request for a communication session submitted by the external device. In particular, US 7,110,823 discloses that the external device sends an RF signal to the implant device to alert it that a communication cycle is desired. The implanted device periodically activates, e.g. once every 1-5 seconds, its RF telemetry receiver for a short period, e.g. during 10-200 milliseconds. If the external device transmits a communication session request during the period in which the receiver is active, the implant device detects the request and a session can be undertaken. The RF telemetry receiver of the implanted device is thus constantly going through a "sleep-listen" cycle when no communication request is detected.

A problem with US 7,110,823 is that the implant device still consumes quite an amount of power, constantly going through its sleep-listen cycles.

WO 2005/099817 discloses a telemetry system involving an implantable medical device and an external device communicating by means of RF. The implantable medical device utilizes a duty cycling for powering up its RF circuitry and the external device initiates a communication session by transmitting a data segment of special wakeup characters.

### Summary of the Invention: The present invention is set out in the appended claims. The embodiments of the present description which do not fall within the scope of said claims are provided for illustrative purposes only and dont fund part of the invention.

An object of the present invention is thus to solve or at least mitigate the above mentioned problems in prior art and to provide a manner of establishing a communication session between a home monitoring device and an IMD in an efficient manner in terms of power consumption at the IMD.

This object is attained by a method of establishing a wireless communication session between an implantable medical device and a home monitoring device in accordance with claim 1 and a system comprising an implantable medical device and a home monitoring device, said system being arranged such that a communication session can be established between the implantable medical device and the home monitoring device in accordance with claim 12.

Preferred embodiments of the invention are defined in the dependent claims.

A basic idea of the invention is to have a home monitoring device frequently send communication-initiating signals to an IMD arranged within the body of a patient for establishing a communication session between the monitoring device and the IMD. In order to save energy, RF telemetry circuitry of the IMD is normally set in a low-power, idle mode. Occasionally, the RF circuitry will leave the low-power mode and enter an active mode in which it is able to detect a communication-initiating signal transmitted by the monitoring device. When a communication-initiating signal is detected, the IMD will respond accordingly to the monitoring device and a communication session will be established. In the present invention, the telemetry circuitry of the IMD will be controlled to wake up from low-power mode and enter an active detection mode *independently* of the transmission of the communication-initiating signals from the monitoring device. Hence, the external monitoring device will frequently transmit communication-initiating signals, but the IMD will only detect a wake-up signal when leaving its low-power mode and entering active mode at a predetermined instant of time.

The present invention is advantageous since the RF circuitry for the most part is set in the low-power mode, in which mode it consumes a negligible amount of power. It should be noted that power consumption is a critical factor in IMD applications. In a practical implementation, the monitoring device may constantly transmit communication-initiating signals, e.g. once every second, while the IMD may enter RF mode once every day and scan the RF channel for communication-initiating signals. Thus, the rate at which the IMD is activated is much lower than the rate at which communication-initiating telemetry signals are transmitted from the home monitoring device. Subsequently, when the IMD has detected a communication-initiating signal, the channel can be established and the monitoring device can read out any data of interest from the IMD. The amount of energy the IMD consumes due to being engaged in a communication session with the monitoring device is thus very small. The energy consumption of the home monitoring device is not a critical operating factor, since the home monitoring device is connected to a mains supply or powered by easily replacable or rechargeable batteries.

In contrast to prior art systems previously discussed, where establishment of communication between an implant device and an external device is effected by means of having the RF telemetry receiver of the implanted device constantly going through sleep-listen cycles, the present invention provides a system in which the monitoring device frequently transmits communication-initiating signals which the IMD occasionally can detect and respond to without constantly having to go through sleep-listen cycles.

The present invention is advantageously implemented in a home environment. In the home environment, it is feasible to transmit communication-initiating signals at a relatively high frequency since it is likely that only a single IMD (or a small number of IMDs) exists that can respond to the numerous communication-initiating signals. Regular monitoring of a patient in an automatic manner may advantageously take place at night, when the patient is asleep and unaware of the actual monitoring. Hence, there is no need for the patient herself to be active for a communication session to be initiated.

In general, the home monitoring device will read out the data from the IMD and terminate the communication session upon completion. When the session is terminated, the IMD will revert to its low-power mode. Possibly, the IMD will confirm to the monitoring device that it has detected a communication-initiating signal before the monitoring device proceeds with the establishment of the communication session.

In an embodiment of the present invention, the transmission of communication-initiating telemetry signals are controlled in such a manner that it is performed in a time interval that overlaps the instant of time at which the implanted medical device is activated from its low-power mode for detection. As a result, the monitoring device will, during a predetermined time interval, frequently transmit wake-up signals which can be detected by the IMD. This embodiment enables a situation where a plurality of home monitoring units can be located in a medical service facility such as a nursing home. The different home monitoring units on the premises of the nursing home can thus be synchronized to efficiently utilize the time and radio spectrum. For instance, a first monitoring unit can be programmed to transmit communication-initiating signals around the time instant when its associated IMD is controlled to wake up from its low-power mode for signal detection, for instance between 10:00 and 10:05 am; a second monitoring unit transmits communication-initiating to its associated IMD between 10:05 and 10:10 am, etc.

Advantageously, the patient in which the IMD is implanted is able to initiate communication by waking the IMD up from low-power mode. For instance, the patient may wake up the IMD by means of a magnet or via communication methods such as inductive telemetry or by using another RF frequency than the one typically utilized when initiating a communication session with the home monitoring device. In practice, the home monitoring device and the IMD may use a 402-405 MHz medical implant communication service (MICS) band for the communication-initiating signal and the subsequent communication session, while a 2.45 GHz industrial, scientific, and medical (ISM) band may be used by the patient to wake up the telemetry circuitry of the IMD up from its low-power mode.

Consequently, the patient does not have to rely on preprogrammed communication-initiating time instants in the IMD for transferring data to the monitoring device. Further, the IMD may be waken up from idle mode due to a specific condition of the patient in which the IMD is implanted, for instance, if the heart rate of the patient falls under, or exceeds, a predetermined level.

In another embodiment of the present invention, the selected instant of time at which the implantable medical device is activated is programmed into the implantable medical device. Further, the rate at which this selected instant of time occurs can be programmed into the implantable medical device. This rate may be programmed to occur randomly.

In a further embodiment of the present invention, if the IMD is activated and a communication-initiating signal is not detected, the implantable medical device is activated again after a predetermined instant of time to check for communication-initiating signal. This will overcome problems which may arise if the IMD cannot be contacted due to temporarily unfavorable conditions of the radio channel. Since the home monitoring device frequently transmits communication-initiating signals, the IMD can assume that it will detected a signal once it wakes up from idle mode.

Further features of, and advantages with, the present invention will become apparent when studying the appended claims and the following description. Those skilled in the art realize that different features of the present invention can be combined to create embodiments other than those explicitly described in the following.

### Brief Description of the Drawings

A detailed description of preferred embodiments of the present invention will be given in the following with reference made to the accompanying drawings, in which:
Fig. 1 is a schematic view of a patient's body showing a pacemaker implanted in the body;
Fig. 2 illustrates functional blocks of an IMD in the form of a pacemaker, to which the present invention may be applied; and
Fig. 3 shows an IMD telemetry device in accordance with an embodiment of the invention.

### Detailed Description of Embodiments of the Invention

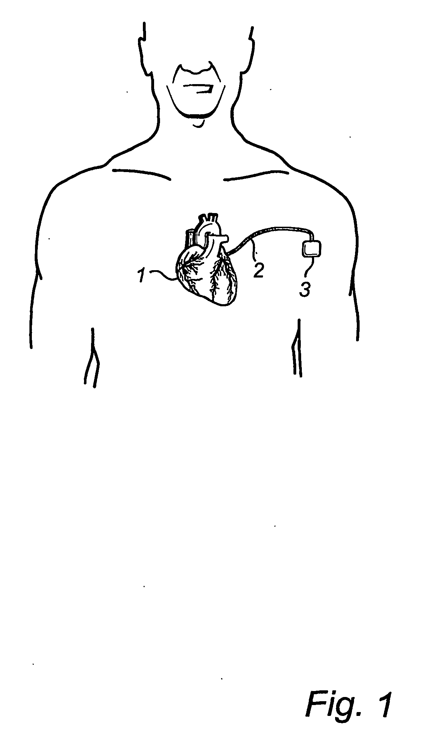

Fig. 1 illustrates an implantable medical device (IMD) in the form of a pacemaker 3 implanted in a patient's body. A lead 2 connects the pacemaker 3 to the heart 1, thereby allowing stimulation of the heart 1 and control of the heart rhythm. In order to transmit and receive RF signals, the pacemaker 3 comprises a transceiver and an antenna (not shown in Fig. 1).

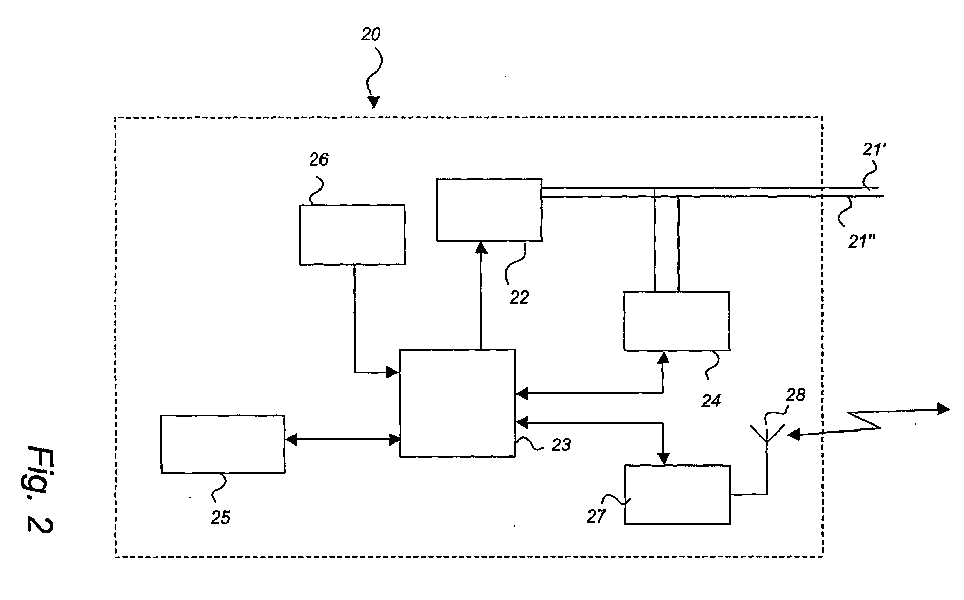

Fig. 2 illustrates functional blocks of a pacemaker 20 (in this particular case a bi-ventricular pacemaker) in more detail. The pacemaker 20 comprises a housing (not shown) being hermetically sealed and biologically inert. Typically, the housing is conductive and may thus serve as an electrode. The pacemaker 20 is connectable to one or more pacemaker leads, where only two are shown in Fig. 2; namely a ventricular lead 21' implanted in the right ventricle of the heart and an atrial lead 21" implanted in the right atrium of the heart. The leads 21', 21" comprise one or more electrodes, such as a tip electrode or a ring electrode, arranged to, inter alia, measure impedance or transmit pacing pulses for causing depolarization of cardiac tissue adjacent to the electrodes generated by a pace pulse generator 22 under influence of a controller or controlling circuit 23 including a microprocessor for e.g. signal processing. The controller 23 controls, inter alia, pace pulse parameters such as output voltage and pulse duration.

Further, storage means 25 is connected to the controller 23, which storage means 25 may include a random access memory (RAM) and/or a non-volatile memory such as a read-only memory (ROM). Detected signals from the patient's heart are processed in an input circuit 24 and are forwarded to the controller 23 for use in logic timing determination in known manner. The pacemaker 20 is powered by a battery 26, which supplies electrical power to all active electrical components of the pacemaker. The pacemaker 20 also comprises an RF transceiver 27 for wireless communication of signals to/from a home monitoring device. Medical personnel or the patient herself may e.g. want to monitor and/or adjust parameters of the pacemaker 20 of to perform reprogramming. The transceiver is connected to an antenna 28 via which the wireless communication occurs. The pacemaker 20 is typically arranged such that it can register an IEGM and provide it to the monitoring device via the RF transceiver 27.

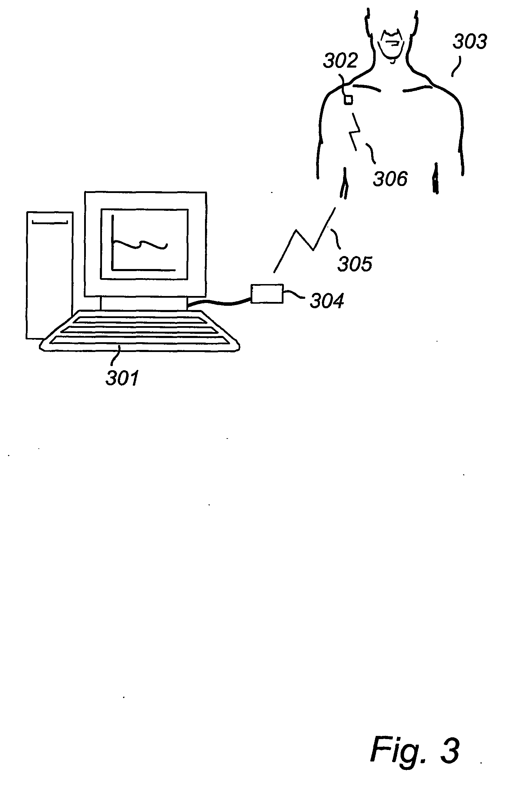

A telemetry system in accordance with an embodiment of the invention is shown in Fig. 3. A home monitoring device 301 is employed to communicate with transceiver circuitry in an IMD 302 implanted into a patient 303 as is described in more detail in Fig. 2. In principle, a home monitoring device is a computer arranged with an RF transceiver 304 connected to the computer via an appropriate connection such as USB, a user interface for controlling the home monitoring device and dedicated software designed for the purpose of communicating with an IMD. Even though not shown in Fig. 3, the RF transceiver 304 may be arranged to conduct wireless transmissions with the monitoring device 301. When establishing communication with an IMD, the home monitoring device broadcasts a communication-initiating signal 305 before a communication session can be undertaken with the concerned IMD. The home monitoring device frequently transmits communication-initiating signals such that the IMD can detect them when waking up from low-power mode.

If the telemetry circuitry of the IMD 302 is activated from its low-power, idle mode, it will detect the communication-initiating signal 305 and reply to the communication-initiating signal by means of transmitting a response signal 306. Then, a communication session can be established between the monitoring device and the IMD. Possibly, the monitoring device terminates the communication session when a requested data set has been received at the monitoring device from the IMD. When the communication session is completed, the IMD reverts to the low-power mode.

However, if the telemetry circuitry of the IMD remains in its low-power mode, no detection of communication-initiating signals will take place, and the monitoring device will proceed with its continuous transmission of signals. Possibly, should the IMD wake up from its low-power mode without detecting a communication-initiating signal, it can be programmed to make further attempts to detect such a signal within a predetermined period of time. This alleviates the situation where the radio channel is inferior and a signal temporarily cannot be detected. A number of parameters may be programmed into the IMD, such as the selected instant of time at which the implantable medical device is activated and the rate at which this selected instant of time occurs. However, in an embodiment of the invention, the transmission of communication-initiating signals are controlled at the monitoring device in such a manner that it is performed in a time interval that overlaps the instant of time at which the implanted medical device is activated from its low-power mode for detection. As a result, the monitoring device will, during a predetermined time interval, frequently transmit wake-up signals which can be detected by the IMD. In such an embodiment, is it not necessary to implement a "fall back" mechanism of making further attempts to detect a communication-initiating signal, as described in the above.

The home monitoring device (and thus an eventual operator of the monitoring device) may be located remotely from the patient 306, for example in another room. It is also possible that signals to be processed by the monitoring device are transmitted to it via a network such as the Internet. As can be seen, the present invention is particularly advantageous in a home environment where it is unlikely that a plurality of monitoring devices are utilizing the same RF band simultaneously. It should be noted that the patient 303 may have a plurality of IMDs implanted with which the monitoring device 301 is able to communicate. A number of situations are possible, for instance
a) *broadcasting*, where any IMD may initiate communication;
b) *pointcasting,* where only one IMD may initiate communication;
c) *multicasting,* where any number of predefined IMDs may initiate communication.

The home monitoring device and its RF transceiver shown throughout the drawings typically employs the 402-405 MHz MICS band for bidirectional communication with the IMD. The MICS standard allows 10 channels, each 300 kHz, to be used in the 402-405 MHz band. Maximum output power is restrained to 25 µW. If the MICS band exclusively is used for communication between the home monitoring device and the IMD, the IMD need not be equipped with 2.45 GHz telemetry circuitry. However, as previously mentioned, the 2.45 GHz band may advantageously be employed by a patient to wake up the IMD from idle mode. In the 2.45 GHz ISM band, it is easy to design a low power reciever that can be used to wake up the device for a MICS communication session. However it is observed that the attenuation of 2.45 GHz signals in body tissue is significant and thus 2.45 GHz wake-up must be done with a short distance between patient and home monitoring device. The MICS band support wireless communication having ranges of 2 meters or more.

The described embodiments are not intended to limit the scope of the invention, as defined by the appended claims.

## Claims

1. A method of establishing a wireless communication session between an implantable medical device and a home monitoring device, the method comprising the steps of:
frequently transmitting communication-initiating telemetry signals from said home monitoring device;
activating telemetry circuitry of the implantable medical device from a low-power mode independently of the transmission of the communication-initiating telemetry signals;
detecting at least one of the communication-initiating telemetry signals at the implantable medical device; and
establishing a communication session between the implantable medical device and the monitoring device, **characterized in that** a rate at which said telemetry circuitry is activated is lower than a rate at which communication-initiating telemetry signals are transmitted.

2. The method according to claim 1, wherein the step of detecting a communication-initiating telemetry signal further comprises the step of:
sending, from the implantable medical device to the monitoring device, a confirmation signal that the communication-initiating telemetry signal was detected.

3. The method according to any one of claims 1 or 2, further comprising the steps of:
terminating, at said monitoring device, the communication session when a requested data set has been received at the monitoring device from the implantable medical device; and
setting the telemetry circuitry of the implantable medical device in the low-power mode when the communication session is terminated.

4. The method according to any one of claims 1-3, wherein the frequent transmission of communication-initiating telemetry signals from said monitoring device are controlled to occur in a time interval that overlaps an instant of time at which the telemetry circuitry of the implanted medical device is activated from its low-power mode for detection.

5. The method according to any one of the preceding claims, wherein a patient in which the medical device is implanted herself can select an instant of time at which the telemetry circuitry of the implantable medical device is activated for detection.

6. The method according to any one of the preceding claims, wherein the selected instant of time at which the telemetry circuitry of the implantable medical device is activated is programmed into the implantable medical device.

7. The method according to any one of the preceding claims, wherein the rate at which the selected instant of time occurs is programmed into the implantable medical device.

8. The method according to claim 7, wherein a random rate for selecting the instant of time is programmed into the implantable medical device.

9. The method according to any one of the preceding claims, wherein, if the telemetry circuitry of the implantable medical device is activated and a communication-initiating telemetry signal is not detected, said telemetry circuitry is activated again after a predetermined instant of time.

10. The method according to any one of the preceding claims, wherein the transmission of communication-initiating telemetry signals and the subsequent communication session is undertaken in the medical implant communication service (MICS) band.

11. The method according to claim 5, wherein the activation of the telemetry circuitry by the patient is undertaken in the industrial, scientific, and medical (ISM) band.

12. A system comprising an implantable medical device (302) and a home monitoring device (301), said system being arranged such that a communication session can be established between the implantable medical device and the home monitoring device, wherein
the home monitoring device is arranged with a transceiver (304) for frequently transmitting communication-initiating telemetry signals to the implantable medical device;
the implantable medical device is arranged with a telemetry circuitry (27) which is configured to be activated from a low-power mode independently of the transmission of the communication-initiating telemetry signals, said telemetry circuitry further being configured to detect at least one of the communication-initiating telemetry signals, wherein a communication session can be established between the implantable medical device and the monitoring device, **characterized in that** a rate at which said telemetry circuitry is activated is lower than a rate at which communication-initiating telemetry signals are transmitted.

13. The system according to claim 12, wherein the implantable medical device (302) is further arranged to send a confirmation signal that the communication-initiating telemetry signal has been detected.

14. The system according to any one of claims 12 or 13, wherein
the home monitoring device (301) is further arranged to terminate the communication session when a requested data set has been received from the implantable medical device; and
the implantable medical device (302) is further arranged with a controller (24) for setting the telemetry circuitry (27) in the low-power mode when the communication session is terminated.

15. The system according to any one of claims 12-14, wherein said home monitoring device (301) is arranged to control the frequent transmission of communication-initiating telemetry signals to occur in a time interval that overlaps an instant of time at which the telemetry circuitry (27) of the implanted medical device (302) is activated from its low-power mode for detection.

16. The system according to any one of claims 12-15, wherein the implantable medical device (302) is arranged with storage means (25) in which the selected instant of time at which the telemetry circuitry (27) is activated and/or the rate at which the selected instant of time occurs can be programmed.

## Patentansprüche

1. Verfahren zum Aufbauen einer Drahtloskommunikationssitzung zwischen einem implantierbaren Medizinprodukt und einem Heimüberwachungsgerät, wobei das Verfahren folgende Schritte umfasst:
häufiges Übertragen kommunikationseinleitender Telemetriesignale von dem Heimüberwachungsgerät aus,
Aktivieren einer Telemetrieschaltung des implantierbaren Medizinprodukts aus einem Niedrigverbrauchsmodus unabhängig von der Übertragung der kommunikationseinleitenden Telemetriesignale,
Erfassen mindestens eins der kommunikationseinleitenden Telemetriesignale an dem implantierbaren Medizinprodukt, und
Aufbauen einer Kommunikationssitzung zwischen dem implantierbaren Medizinprodukt und dem Überwachungsgerät, **dadurch gekennzeichnet, dass** eine Geschwindigkeit, mit der die Telemetrieschaltung aktiviert wird, geringer ist als eine Geschwindigkeit, mit der kommunikationseinleitende Telemetriesignale übertragen werden.

2. Verfahren nach Anspruch 1, wobei der Schritt des Erfassens eines kommunikationseinleitenden Telemetriesignals ferner folgenden Schritt umfasst:
Senden, von dem implantierbaren Medizinprodukt zum Überwachungsgerät, eines Bestätigungssignals, dass das kommunikationseinleitende Telemetriesignal erfasst wurde.

3. Verfahren nach einem der Ansprüche 1 oder 2, ferner umfassend die folgenden Schritte:
Beenden, an dem Überwachungsgerät, der Kommunikationssitzung, wenn ein angefragter Datensatz an dem Überwachungsgerät von dem implantierbaren Medizinprodukt aus empfangen wurde, und
Versetzen der Telemetrieschaltung des implantierbaren Medizinprodukts in den Niedrigverbrauchsmodus, wenn die Kommunikationssitzung beendet ist.

4. Verfahren nach einem der Ansprüche 1 bis 3, wobei die häufige Übertragung kommunikationseinleitender Telemetriesignale von dem Überwachungsgerät aus gesteuert wird, damit sie in einem Zeitraum erfolgt, der sich mit einem Zeitpunkt überschneidet, zu dem die Telemetrieschaltung des implantierten Medizinprodukts aus ihrem Niedrigverbrauchsmodus zur Erfassung aktiviert wird.

5. Verfahren nach einem der vorhergehenden Ansprüche, wobei ein Patient, in den das Medizinprodukt implantiert ist, selbst einen Zeitpunkt wählen kann, zu dem die Telemetrieschaltung des implantierbaren Medizinprodukts zur Erfassung aktiviert wird.

6. Verfahren nach einem der vorhergehenden Ansprüche, wobei der gewählte Zeitpunkt, zu dem die Telemetrieschaltung des implantierbaren Medizinprodukts aktiviert wird, in das implantierbare Medizinprodukt programmiert wird.

7. Verfahren nach einem der vorhergehenden Ansprüche, wobei die Geschwindigkeit, mit der der gewählte Zeitpunkt eintritt, in das implantierbare Medizinprodukt programmiert wird.

8. Verfahren nach Anspruch 7, wobei eine zufällige Geschwindigkeit für das Wählen des Zeitpunkts in das implantierbare Medizinprodukt programmiert wird.

9. Verfahren nach einem der vorhergehenden Ansprüche, wobei, wenn die Telemetrieschaltung des implantierbaren Medizinprodukts aktiviert wird und kein kommunikationseinleitendes Telemetriesignal erfasst wird, die Telemetrieschaltung nach einem bestimmten Zeitpunkt erneut aktiviert wird.

10. Verfahren nach einem der vorhergehenden Ansprüche, wobei die Übertragung kommunikationseinleitender Telemetriesignale und die anschließende Kommunikationssitzung im MICS-Band (MICS - Medical Implant Communication Service) vorgenommen werden.

11. Verfahren nach Anspruch 5, wobei die Aktivierung der Telemetrieschaltung durch den Patienten im ISM-Band (ISM - Industrial, Scientific and Medical) vorgenommen wird.

12. System, umfassend ein implantierbares Medizinprodukt (302) und ein Heimüberwachungsgerät (301), wobei das System derart angeordnet ist, dass eine Kommunikationssitzung zwischen dem implantierbaren Medizinprodukt und dem Heimüberwachungsgerät aufgebaut werden kann, wobei
das Heimüberwachungsgerät mit einem Transceiver (304) zum häufigen Übertragen kommunikationseinleitender Telemetriesignale an das implantierbare Medizinprodukt ausgestattet ist,
das implantierbare Medizinprodukt mit einer Telemetrieschaltung (27) ausgestattet ist, die eingerichtet ist, unabhängig von der Übertragung der kommunikationseinleitenden Telemetriesignale aus einem Niedrigverbrauchsmodus aktiviert zu werden, wobei die Telemetrieschaltung ferner eingerichtet ist, mindestens eins der kommunikationseinleitenden Telemetriesignale zu erfassen, wobei eine Kommunikationssitzung zwischen dem implantierbaren Medizinprodukt und dem Überwachungsgerät aufgebaut werden kann, **dadurch gekennzeichnet, dass** eine Geschwindigkeit, mit der die Telemetrieschaltung aktiviert wird, geringer ist als eine Geschwindigkeit, mit der kommunikationseinleitende Telemetriesignale übertragen werden.

13. System nach Anspruch 12, wobei das implantierbare Medizinprodukt (302) ferner angeordnet ist, ein Bestätigungssignal zu senden, dass das kommunikationseinleitende Telemetriesignal erfasst wurde.

14. System nach einem der Ansprüche 12 oder 13, wobei
das Heimüberwachungsgerät (301) ferner angeordnet ist, die Kommunikationssitzung zu beenden, wenn ein angefragter Datensatz von dem implantierbaren Medizinprodukt aus empfangen wurde, und
das implantierbare Medizinprodukt (302) ferner mit einer Steuerung (24) ausgestattet ist, um die Telemetrieschaltung (27) in den Niedrigverbrauchsmodus zu versetzen, wenn die Kommunikationssitzung beendet ist.

15. System nach einem der Ansprüche 12 bis 14, wobei das Heimüberwachungsgerät (301) angeordnet ist, die häufige Übertragung kommunikationseinleitender Telemetriesignale zu steuern, damit sie in einem Zeitraum erfolgt, der sich mit einem Zeitpunkt überschneidet, zu dem die Telemetrieschaltung (27) des implantierten Medizinprodukts (302) aus ihrem Niedrigverbrauchsmodus zur Erfassung aktiviert wird.

16. System nach einem der Ansprüche 12 bis 15, wobei das implantierbare Medizinprodukt (302) mit Speichermitteln (25) ausgestattet ist, in denen der gewählte Zeitpunkt, zu dem die Telemetrieschaltung (27) aktiviert wird, und/oder die Geschwindigkeit, mit der der gewählte Zeitpunkt eintritt, programmiert sein kann bzw. können.

## Revendications

1. Procédé d'établissement d'une session de communication sans fil entre un dispositif médical implantable et un dispositif de surveillance domestique, le procédé comprenant les étapes consistant à :
transmettre fréquemment des signaux de télémesure initialisant une communication, à partir dudit dispositif de surveillance domestique ;
activer des circuits de télémesure du dispositif médical implantable à partir d'un mode à faible consommation indépendamment de la transmission des signaux de télémesure initialisant une communication ;
détecter au moins un des signaux de télémesure initialisant une communication sur le dispositif médical implantable ; et
établir une session de communication entre le dispositif médical implantable et le dispositif de surveillance, **caractérisé en ce qu'**une vitesse à laquelle lesdits circuits de télémesure sont activés est inférieure à une vitesse à laquelle des signaux de télémesure initialisant une communication sont transmis.

2. Procédé selon la revendication 1, dans lequel l'étape de détection d'un signal de télémesure initialisant une communication comprend en outre l'étape consistant à :
envoyer, du dispositif médical implantable au dispositif de surveillance, un signal de confirmation selon lequel le signal de télémesure initialisant une communication a été détecté.

3. Procédé selon l'une quelconque des revendications 1 ou 2, comprenant en outre les étapes consistant à :
terminer, sur ledit dispositif de surveillance, la session de communication quand un jeu de données requis a été reçu sur le dispositif de surveillance en provenance du dispositif médical implantable ; et
mettre les circuits de télémesure du dispositif médical implantable dans le mode à faible consommation quand la session de communication est terminée.

4. Procédé selon l'une quelconque des revendications 1 à 3, dans lequel la transmission fréquente de signaux de télémesure initialisant une communication en provenance dudit dispositif de surveillance est contrôlée pour se produire dans un intervalle de temps qui chevauche un instant auquel les circuits de télémesure du dispositif médical implanté sont activés à partir de leur mode à faible consommation pour la détection.

5. Procédé selon l'une quelconque des revendications précédentes, dans lequel un patient dans lequel un dispositif médical est implanté peut sélectionner lui-même un instant auquel les circuits de télémesure du dispositif médical implantable sont activés pour la détection.

6. Procédé selon l'une quelconque des revendications précédentes, dans lequel l'instant sélectionné auquel les circuits de télémesure du dispositif médical implantable sont activés est programmé dans le dispositif médical implantable.

7. Procédé selon l'une quelconque des revendications précédentes, dans lequel la vitesse à laquelle l'instant sélectionné se produit est programmée dans le dispositif médical implantable.

8. Procédé selon la revendication 7, dans lequel une vitesse aléatoire pour sélectionner l'instant est programmée dans le dispositif médical implantable.

9. Procédé selon l'une quelconque des revendications précédentes, dans lequel, si les circuits de télémesure du dispositif médical implantable sont activés et qu'un signal de télémesure initialisant une communication n'est pas détecté, lesdits circuits de télémesure sont réactivés après un instant prédéterminé.

10. Procédé selon l'une quelconque des revendications précédentes, dans lequel la transmission de signaux de télémesure initialisant une communication et la session de communication subséquente sont effectuées dans la bande du service de communication des implants médicaux (Medical Implant Communication Service, MICS).

11. Procédé selon la revendication 5, dans lequel l'activation des circuits de télémesure par le patient est effectuée dans la bande industrielle, scientifique et médicale (ISM).

12. Système comprenant un dispositif médical implantable (302) et un dispositif de surveillance domestique (301), ledit système étant agencé de sorte qu'une session de communication peut être établie entre le dispositif médical implantable et le dispositif de surveillance domestique, dans lequel
le dispositif de surveillance domestique est agencé avec un émetteur-récepteur (304) pour transmettre fréquemment des signaux de télémesure initialisant une communication au dispositif médical implantable ;
le dispositif médical implantable est agencé avec des circuits de télémesure (27) qui sont configurés pour être activés à partir d'un mode à faible consommation indépendamment de la transmission des signaux de télémesure initialisant une communication, lesdits circuits de télémesure étant en outre configurés pour détecter au moins un des signaux de télémesure initialisant une communication, dans lequel une session de communication peut être établie entre le dispositif médical implantable et le dispositif de surveillance, **caractérisé en ce qu'**une vitesse à laquelle lesdits circuits de télémesure sont activés est inférieure à une vitesse à laquelle des signaux de télémesure initialisant une communication sont transmis.

13. Système selon la revendication 12, dans lequel le dispositif médical implantable (302) est en outre agencé pour envoyer un signal de confirmation selon lequel le signal de télémesure initialisant une communication a été détecté.

14. Système selon l'une quelconque des revendications 12 ou 13, dans lequel
le dispositif de surveillance domestique (301) est en outre agencé pour terminer la session de communication quand un jeu de données requis a été reçu en provenance du dispositif médical implantable ; et
le dispositif médical implantable (302) est en outre agencé avec un contrôleur (24) pour mettre les circuits de télémesure (27) dans le mode à faible consommation quand la session de communication est terminée.

15. Système selon l'une quelconque des revendications 12 à 14, dans lequel ledit dispositif de surveillance domestique (301) est agencé pour contrôler la transmission fréquente de signaux de télémesure initialisant une communication de façon à ce qu'elle se produise dans un intervalle de temps qui chevauche un instant auquel les circuits de télémesure (27) du dispositif médical implantable (302) sont activés à partir de leur mode à faible consommation pour la détection.

16. Système selon l'une quelconque des revendications 12 à 15, dans lequel le dispositif médical implantable (302) est agencé avec des moyens de stockage (25) dans lesquels l'instant sélectionné auquel les circuits de télémesure (27) sont activés et/ou la vitesse à laquelle l'instant sélectionné se produit peuvent être programmés.
